**Europäisches Patentamt**

**European Patent Office**

**Office européen des brevets**

(19)

(11) Numéro de publication : **0 356 282 B1**

(12) # FASCICULE DE BREVET EUROPEEN

(45) Date de publication du fascicule du brevet :
**27.12.91 Bulletin 91/52**

(51) Int. Cl.$^5$ : **A61F 9/00**

(21) Numéro de dépôt : **89402158.3**

(22) Date de dépôt : **28.07.89**

(54) Dispositif à laser, notamment pour applications thérapeutiques.

(30) Priorité : **02.08.88 FR 8810416**

(43) Date de publication de la demande :
**28.02.90 Bulletin 90/09**

(45) Mention de la délivrance du brevet :
**27.12.91 Bulletin 91/52**

(84) Etats contractants désignés :
**AT BE CH DE ES GB GR IT LI LU NL SE**

(56) Documents cités :
**EP-A- 0 007 256**
**DE-A- 3 518 256**
**GB-A- 2 074 343**

(73) Titulaire : **ALCON PHARMACEUTICALS
LIMITED**
**Sinserstrasse 47**
**CH-6330 Cham (CH)**

(72) Inventeur : **Dacquay, Bruno**
**9, rue A. Varenne**
**F-63210 Beaumont (FR)**
Inventeur : **Chaduc, Jean-Paul**
**162, rue du Courage**
**F-63000 Clermont-Ferrand (FR)**

(74) Mandataire : **Bonnetat, Christian**
**CABINET BONNETAT 23, Rue de Léningrad**
**F-75008 Paris (FR)**

## Description

La présente invention concerne un dispositif à laser du type comportant un laser de puissance destiné à émettre un rayonnement dont la longueur d'onde est située dans le domaine spectral visible.

Dans une application préférentielle, quoique non exclusive, le dispositif à laser selon l'invention est plus particulièrement approprié à une utilisation thérapeutique, telle que, par exemple, en ophtalmologie, bien que d'autres applications, notamment industrielles, pourraient être envisagées.

Les dispositifs à laser employés, par exemple, dans le domaine de l'ophtalmologie peuvent être dissociés en deux groupes qui sont fonction de la longueur d'onde du rayonnement émis.

Le premier groupe concerne les dispositifs à laser comprenant un laser de puissance dont la longueur d'onde du rayonnement susceptible d'être émis se situe dans le domaine spectral visible, tandis que le second groupe concerne ceux dont le laser de puissance est susceptible d'émettre un rayonnement de longueur d'onde située dans le domaine spectral non visible.

Dans ce second groupe, pour que le praticien puisse effectuer, par exemple, le traitement de la région déficiente de l'oeil par le rayonnement non visible émis par le laser de puissance, les dispositifs comportent de plus un laser de visée auxiliaire. Ce dernier émet un rayonnement dans le domaine spectral visible, ce qui permet ainsi au praticien de régler la position, sur la région de l'oeil à traiter, du point d'impact du rayonnement visible émis par le laser auxiliaire et sur lequel est amené en coïncidence le rayonnement non visible du laser de puissance destiné au traitement proprement dit.

Malgré la présence du laser de visée auxiliaire, ces dispositifs, dans leurs applications médicales, font l'objet de réserves de la part des praticiens, du fait que le rayonnement du laser de puissance, destiné au traitement, est invisible

Quant aux dispositifs à laser du premier groupe, le rayonnement visible sortant du laser de puissance, par exemple, du type argon, sert à la fois de traitement et de visée. Toutefois, lorsque le praticien procède à la visée de la région de l'oeil à traiter, la puissance du rayonnement sortant du laser doit être diminuée.

Pour cela, le dispositif à laser comporte un atténuateur disposé en sortie du laser de puissance et permettant, lorsqu'il est placé dans le trajet du rayonnement, de diminuer la puissance du faisceau disponible. Par ailleurs, un filtre mobile doit être également prévu devant les yeux du praticien pour éviter que ceux-ci ne soient exposés au rayonnement de puissance dirigé vers la région de l'oeil du patient à traiter. Ainsi, lorsque l'atténuateur est placé sur le trajet du rayonnement émis sortant du laser de puissance, le filtre mobile est escamoté, puisque la puissance dudit rayonnement est alors diminuée, le praticien pouvant alors procéder au réglage de la visée du rayonnement modéré en direction de la région déficiente de l'oeil à traiter. En revanche, lorsque l'atténuateur est escamoté, le filtre mobile est disposé dans le champ de vision du praticien en faisant ainsi écran de protection, puisque la puissance du rayonnement émis est alors maximale. Le praticien peut alors procéder aux "tirs" destinés au traitement de ladite région de l'oeil.

Ces dispositifs à laser, très répandus, présentent toutefois des inconvénients.

Tout d'abord, le temps d'utilisation du laser de puissance à argon est imparti environ pour 99% à la visée, alors que le temps consacré au traitement ne correspond qu'à 1% du temps d'utilisation dudit laser.

Il ressort donc que la durée d'utilisation du laser à argon est pratiquement consacrée à la visée. Cela implique, outre une durée de vie réduite du laser, un système de refroidissement complexe de ce dernier, puisque le laser fonctionne pleinement pendant les temps de visée et de traitement, qui, généralement, durent respectivement 10 secondes et 0,1 seconde par tir.

De plus, comme on l'a rappelé précédemment, un tel dispositif impose la présence d'un atténuateur et d'un filtre escamotables.

La présente invention a pour but d'obvier aux inconvénients des dispositifs antérieurs, et, concerne un dispositif à laser du type comportant au moins un laser de puissance susceptible d'émettre un rayonnement de longueur d'onde visible, le temps d'utilisation dudit laser de puissance étant uniquement consacré au traitement proprement dit, tel que, par exemple, dans l'application précitée, de la région d'un oeil, tout en assurant que ledit rayonnement du laser de puissance est exactement dirigé sur ladite cible, en évitant, de plus, la présence d'un système de refroidissement complexe dudit laser, d'un atténuateur et d'un filtre escamotables.

A cet effet, le dispositif à laser, du type comportant au moins un laser de puissance susceptible d'émettre un rayonnement de longueur d'onde située dans le domaine spectral visible, destiné à produire une action sur une une cible, au moins un laser de visée auxiliaire susceptible d'émettre un rayonnement de longueur d'onde située dans le domaine spectral visible, dirigé sur une sone de ladite cible sur laquelle peut agir le rayonnement émis par ledit laser de puissance, les rayonnements émis respectivement par le laser de puissance et le laser de visée suivant un trajet optique commun passant par un dispositif optique mobile par rapport à ladite cible, est caractérisé en ce qu'il comporte un filtre associé audit dispositif optique et permettant de protéger l'observateur quand le rayonnement de puissance est déclenché, et ainsi lui permettant de le visualiser également.

Ainsi, grâce à l'invention, l'utilisation d'un laser de visée auxiliaire émettant un rayonnement visible permet au laser de puissance, émettant également un rayonnement visible, de ne fonctionner que durant le traitement proprement dit, ce qui augmente considérablement sa durée de vie et sa fréquence d'utilisation. Par ailleurs, il en découle que le système de refroidissement du laser de puissance est beaucoup plus simple puisque son temps de fonctionnement est diminué, par exemple, d'un facteur 100 pour l'application médicale précitée d'un laser à argon.

Aussi, la présence d'un atténuateur sur le trajet du rayonnement émis par le laser de puissance est inutile, ce dernier n'étant déclenché que pour le traitement.

On remarquera que le brevet GB-A-2074343 concerne un dispositif à laser comportant un laser de puissance à rayonnement visible, associé à un laser de visée auxiliaire. En réalité, cette particularité n'est utilisée nulle part dans le dispositif décrit dans ce brevet antérieur et, par ailleurs, l'objet principal de ce brevet antérieur est un dispositif d'expansion transversale du faisceau de puissance. En d'autres termes, si aucune référence n'était faite à la propriété visible du rayonnement du laser de puissance, rien se serait changé dans ce qui est décrit dans le document antérieur.

D'ailleurs, le dispositif de ce brevet britannique antérieur comporte un obturateur qui isole les yeux du chirurgien du faisceau de puissance pendant les tirs laser. Ainsi, pendant l'opération, le chirurgien est incapable de visualiser le point d'impact exact du laser de puissance dont la propriété du faisceau visible ne peut donc être utilisée. On retrouve là les inconvénients mentionnés ci-dessus à propos des dispositifs du premier et du second groupe de dispositifs à laser mentionnés.

De préférence, ledit laser de puissance est un laser à argon, tandis que le laser de visée auxiliaire peut être un laser à hélium-néon. Ainsi, les deux lasers émettent des rayonnements visibles distincts.

Selon une autre caractéristique de l'invention, lesdits rayonnements émis par le laser de puissance et par le laser de visée auxiliaire sont reçus par un coupleur qui permet l'alignement desdits rayonnements selon ledit trajet optique commun. Dans un mode préféré de réalisation, lesdits rayonnements alignés se propagent dans une fibre optique qui débouche dans ledit dispositif optique.

Le dispositif optique peut alors comporter un organe optique de puissance recevant et transmettant lesdits rayonnements, un appareil optique de visée et un miroir réglable recevant les rayonnements transmis par ledit organe optique, et les dirigeant vers ladite cible.

Dans un mode préféré de réalisation, ledit organe de puissance est un zoom, et ledit appareil optique de visée peut être un microscope auquel est rapporté ledit filtre destiné à protéger l'observateur quand le rayonnement de puissance est déclenché. Le filtre peut alors, avantageusement, être fixé à demeure audit microscope, en étant placé dans le champ de vision de l'observateur.

Selon une autre caractéristique, un obturateur peut être interposé entre le laser de puissance et ledit coupleur, sur le trajet du rayonnement émis par ledit laser.

Les figures du dessin annexé feront bien comprendre comment l'invention peut être réalisée. Sur ces figures, des références identiques désignent des éléments semblables.

Les figures 1A et 1B représentent schématiquement un dispositif à laser selon l'art antérieur, appliqué au domaine du traitement des yeux, et, montrent respectivement la phase correspondant à la visée de la région de l'oeil concernée et la phase correspondant au traitement proprement dit de ladite région.

La figure 2 représente schématiquement le dispositif à laser selon l'invention appliqué au même domaine.

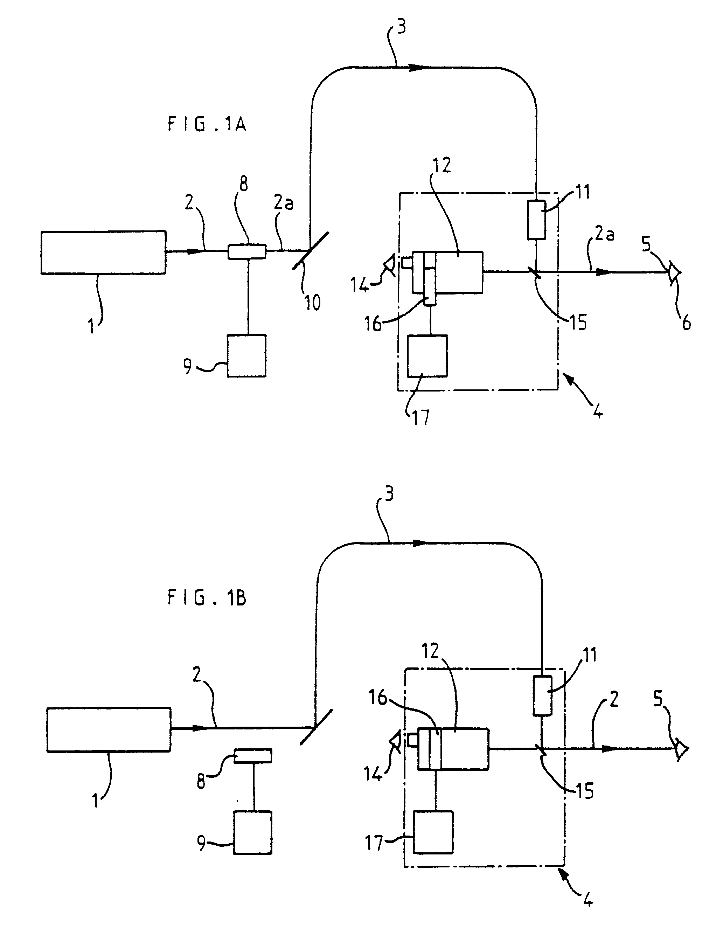

Dans cette application, le dispositif à laser selon l'art antérieur montré sur les figures 1A et 1B comprend un laser 1, par exemple, à argon, susceptible d'émettre un rayonnement 2 de longueur d'onde située dans le domaine spectral visible, ledit rayonnement étant destiné à être dirigé, par l'intermédiaire notamment d'une fibre optique 3 et d'un dispositif optique 4, vers la région 5 de la zone de l'oeil 6 d'un patient destinée à être traitée.

Dans ce dispositif à laser, la puissance du rayonnement émis 2 par le laser à argon 1 doit nécessairement être réduite, lorsque l'observateur ou praticien procède au positionnement, sur la région 5 de l'oeil du patient, du point d'impact du faisceau visible correspondant au rayonnement 2 émis par le laser 1. Pour cela, comme le montre la figure 1A, un atténuateur escamotable 8, relié à un organe de commande 9, est interposé sur le trajet du rayonnement émis 2, entre le laser 1 et un réflecteur 10 permettant d'envoyer le rayonnement 2a, alors diminué en puissance, dans la fibre optique 3.

Le rayonnement 2a débouche dans le dispositif optique 4 par l'extrémité de la fibre optique 3 équipée d'un organe optique de puissance 11, tel que, par exemple, un zoom. Ce dispositif 4 comprend, outre l'organe optique 11, un appareil optique de visée 12, tel que, par exemple, un microscope, les yeux 14 du praticien ayant été représentés en regard du microscope 12. Un miroir réglable 15 est également prévu, de préférence, à l'intersection du rayonnement sortant de la fibre 3 et du champ de visée du microscope 12 et permet de réfléchir le rayonnement 2a qui, dans le cas de la figure 1A, aboutit à la région de l'oeil à traiter du patient.

On remarque, par ailleurs, que le microscope 12 est muni d'un filtre escamotable 16, relié à un organe de commande 17. Sur cette figure 1A, le filtre 16 est dans la position escamotée puisque le rayonnement 2a émis et sortant de la fibre 3 présente une puissance diminuée en raison de l'atténuateur 8.

Le praticien peut ainsi procéder au réglage de la position du point d'impact du faisceau visible, correspondant au rayonnement 2a, sur la région 5 à traiter de l'oeil 6 du patient.

A ce moment, le praticien peut procéder au traitement proprement dit de ladite région 5. Pour cela, il actionne l'organe de commande 17 qui entraîne la mise en place du filtre 16 dans le champ de vision du praticien, puis, l'organe de commande 9 escamote l'atténuateur 8 du trajet du rayonnement 2 émis par le laser 1. Lors du ou des tirs, la protection est généralement totale, de sorte que le praticien ne peut visualiser le point d'impact du rayonnement de puissance 2 sur la région 5 de l'oeil à traiter.

Les inconvénients d'un tel dispositif décrit en regard des figures 1A et 1B ont été énumérés préalablement, et concernent brièvement, d'une part, la durée d'utilisation du laser 1 qui fonctionne en pleine puissance aussi bien pendant le temps de visée (environ 10 secondes) que pendant le temps de traitement (0,1 seconde) pour chaque tir, et d'autre part, l'agencement d'un atténuateur et d'un filtre tous deux commandables entre deux positions distinctes de fonctionnement. Par ailleurs, puisque le laser fonctionne pendant un long moment, le système de refroidissement de celui-ci, bien que non représenté sur les figures 1A et 1B est techniquement complexe.

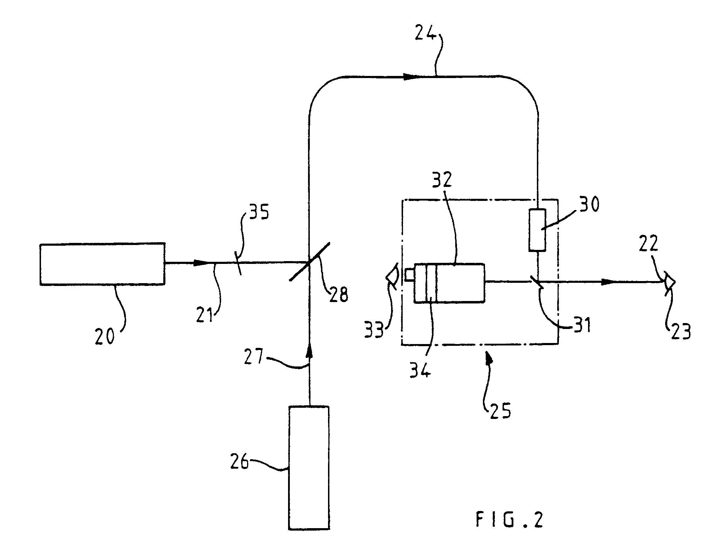

En revanche, le dispositif à laser selon l'invention montré schématiquement sur la figure 2 pallie ces inconvénients. Pour cela, il comporte un laser de puissance 20, tel qu'un laser à argon, susceptible d'émettre un rayonnement 21 de longueur d'onde située dans le domaine spectral visible, ledit rayonnement étant destiné au traitement de la région 22 d'un oeil 23 d'un patient par l'intermédiaire dans cet exemple de réalisation, d'une fibre optique 24 et d'un dispositif optique 25.

Le dispositif à laser comporte, de plus, un laser de visée auxiliaire 26, tel qu'un laser à hélium-néon, susceptible d'émettre un rayonnement 27 de longueur d'onde située dans le domaine spectral visible, ledit rayonnement 27 étant destiné à la visée de ladite région 22.

Par exemple, les longueurs d'onde du rayonnement 21 émis par le laser de puissance 20 sont notamment de 488 et de 514 nanomètres, correspondant à un rayonnement de couleur bleu-vert, tandis que la longueur d'onde du rayonnement 27 émis par le laser de visée auxiliaire 26 est d'environ 632 nanomètres, correspondant à un rayonnement de couleur rouge.

Par ailleurs, les rayonnements 21 et 27, respectivement du laser de puissance 20 et du laser de visée 26, sont reçus par un coupleur 28 permettant l'alignement desdits rayonnements suivant un trajet optique commun, la fibre optique 24 véhiculant alors lesdits rayonnements.

L'extrémité de la fibre optique 24 débouche dans le dispositif optique 25 et comporte un organe de puissance 30 tel que, par exemple, un zoom ou analogue. Les rayonnements traversant ledit zoom sont reçus par un miroir réglable 31 qui permet d'orienter le rayonnement 27 de visée en direction de la région 22 à traiter de l'oeil, ledit rayonnement de puissance 21 suivant le même chemin optique que le rayonnement 27 puisque ces rayonnements se propagent dans une même fibre optique.

Le dispositif optique 25 comporte également un appareil de visée 32, tel qu'un microscope, grâce auquel les yeux 33 du praticien peuvent observer les rayonnements transmis. Un filtre 34 est alors prévu dans le champ de vision du microscope et permet ainsi au praticien de pouvoir visualiser indifféremment, en toute protection, les rayonnements émis. Grâce au filtre, le praticien peut alors visualiser le rayonnement émis par le laser de visée et/ou par le laser de puissance. Il peut donc contrôler le point d'impact du rayonnement de puissance sur la région de l'oeil à traiter par rapport à celui du rayonnement de visée pendant le traitement. Ce filtre 34 peut alors avantageusement être fixé au microscope. Le miroir réglable 31 présente une petite dimension, de sorte que le champ de vision du microscope, au centre duquel est disposé ledit miroir, n'est pratiquement pas réduit ce qui permet au praticien de visualiser correctement lesdits rayonnements en direction de la cible correspondant à la région 22 de l'oeil du patient.

Par ailleurs, il est également prévu, entre le laser de puissance 20 et le coupleur 28, un obturateur 35 situé sur le trajet du rayonnement 21 émis par le laser 20, ledit obturateur évitant d'envoyer au patient les variations d'amplitude du rayonnement dues au temps de montée du laser 20.

Le fonctionnement du dispositif à laser se déroule de la façon suivante : Le praticien déclenche le laser de visée auxiliaire 26 dont le rayonnement émis 27 parvient au miroir réglable 31 du dispositif optique 25, après avoir traversé le coupleur 28, la fibre optique 24 et le zoom 30. Le praticien, qui observe par le microscope 32, peut alors agir sur le miroir réglable 31 pour orienter, de façon appropriée, le rayonnement 27 en direction de la région 22 à traiter de l'oeil du patient.

Lorsque le rayonnement 27 cible correctement la région 22, le praticien déclenche alors le laser de puissance 20, dont le rayonnement émis 21, aligné par rapport au rayonnement de visée 27, atteint ladite région 22 après avoir été réfléchi par le miroir 31 en direction de ladite région. Le praticien peut alors procéder à un ou plusieurs "tirs" pour traiter la région 22 de l'oeil, chaque tir durant approximativement 0,1 seconde.

Par conséquent, puisque la visée est réalisée au moyen du laser auxiliaire 26, il ressort que le laser de puissance fonctionne exclusivement durant le temps de traitement proprement dit, ce qui, par rapport au dispositif à laser décrit en regard des figures 1A et 1B, permet d'augmenter considérablement la durée d'utilisation du laser à argon et donc sa durée de vie.

De plus, avec le dispositif à laser selon l'invention, il n'est plus nécessaire d'avoir à la fois un atténuateur de puissance du rayonnement émis et un filtre mobile. Selon un autre avantage dudit dispositif de l'invention, puisque le laser de puissance ne fonctionne que pendant la durée du traitement, soit 0,1 seconde pour chaque tir, il n'est pas nécessaire d'avoir un système de refroidissement complexe et élaboré de celui-ci, comme c'était le cas pour le dispositif antérieur.

## Revendications

1. Dispositif à laser du type comportant au moins un laser de puissance (20) susceptible d'émettre un rayonnement de longueur d'onde située dans le domaine spectral visible, destiné à produire une action sur une cible, au moins un laser de visée auxiliaire (26) susceptible d'émettre un rayonnement (27) de longueur d'onde située dans le domaine spectral visible, dirigé sur une sone de ladite cible sur laquelle peut agir le rayonnement (21) émis par ledit laser de puissance (20), les rayonnements émis respectivement par le laser de puissance et le laser de visée suivant un trajet optique commun passant par un dispositif optique (25) mobile par rapport à ladite cible, caractérisé en ce qu'il comporte un filtre (34) associé audit dispositif optique (25) et permettant de protéger l'observateur quand le rayonnement de puissance (21) est déclenché, et ainsi lui permettant de la visualiser également.

2. Dispositif selon la revendication 1, caractérisé en ce que ledit laser de puissance (20) est un laser à argon.

3. Dispositif selon l'une des revendications 1 ou 2, caractérisé en ce que ledit laser de visée auxiliaire (26) est un laser à hélium-néon.

4. Dispositif selon l'une quelconque des revendications 1 à 3, caractérisé en ce que lesdits rayonnements émis par le laser de puissance (20) et par le laser de visée auxiliaire (26) sont reçus par un coupleur (28) qui permet l'alignement desdits rayonnements selon ledit trajet optique commun.

5. Dispositif selon l'une quelconque des revendications 1 à 4, caractérisé en ce que lesdits rayonnements alignés se propagent dans une fibre optique (24) qui débouche dans ledit dispositif optique (25).

6. Dispositif selon l'une quelconque des revendications 1 à 5, caractérisé en ce que ledit dispositif optique (25) comporte un organe optique de puissance (30) recevant et transmettant lesdits rayonnements, un appareil optique de visée (32) et un miroir réglable (31) recevant les rayonnements transmis par ledit organe optique et les dirigeant vers ladite cible.

7. Dispositif selon la revendication 6, caractérisé en ce que ledit organe optique de puissance (30) est un zoom.

8. Dispositif selon l'une des revendications 6 ou 7, caractérisé en ce que ledit appareil optique de visée (32) est un microscope auquel est rapporté ledit filtre (34) destiné à protéger l'observateur quand le rayonnement de puissance (21) est déclenché.

9. Dispositif selon la revendication 8, caractérisé en ce que ledit filtre (34) est fixé à demeure audit microscope (32), en étant placé dans le champ de vision de l'observateur.

10. Dispositif selon l'une quelconque des revendications 4 à 9, caractérisé en ce qu'un obturateur (35) est interposé entre le laser de puissance (20) et ledit coupleur (28), sur le trajet du rayonnaient émis par ledit laser.

## Patentansprüche

1. Lasereinrichtunp, einer Bauart, bestehend aus einem leistungsfähigen Laser (20), der geeignet ist, einen Strahl mit einer im Bereich des sichtbaren Spektrums liegenden Wellenlänge auszusenden, der dazu bestimmt ist, Wirkung auf einen Zielbereich auszuüben, wenigstens einem Neben- Ziellaser (26), der geeignet ist, einen Strahl (27) mit einer im Bereich des sichtbaren Spektrums liegenden Wellenlänge auszusenden, der in den Zielbereich geleitet wird, auf welchen der durch den leistungsfähigen Laser (20) ausgesendete Strahl (21) einwirkt, wobei die jeweils von dem leistungsfähigen Laser und dem Neben- Ziellaser ausgesendeten Strahlen entlang einer gemeinsamen optischen Bahn eine optische Einrichtung (25), die in Bezug auf den Zielbereich beweglich ist, durchfahren, dadurch gekennzeichnet, daß ein Filter (34), der es gestattet, den Beobachter zu schützen, wenn der leistungsfähige Strahl (21) ausgelöst ist, mit der optischen Einrichtung (25) verbunden ist und den Zielbereich gleichfalls sichtbar macht.

2. Einrichtung nach Anspruch 1, dadurch gekennzeichnet, daß der leistungsfähige Laser (20) ein Argon- Laser ist.

3. Einrichtung nach Anspruch 1 oder 2, dadurch gekennzeichnet, daß der Neben- Ziellaser (26) ein Helium- Neon- Laser ist.

4. Einrichtung nach einem der Ansprüche 1-3, dadurch gekennzeichnet, daß die durch den leistungsfähigen Laser (20) und durch den Neben- Ziellaser (26) ausgesendeten Strahlen über eine Kupplung (28), welche eine Gleichrichtung der Strahlen gemäß der gemeinsamen optischen Bahn ermöglicht, erhältlich sind.

5. Einrichtung nach einem der Ansprüche 1-4, dadurch gekennzeichnet, daß die gleichgerichteten Strahlen eine in die optische Einrichtung (25) mündende optische Faser (24) durchströmen.

6. Einrichtung nach einem der Ansprüche 1-5, dadurch gekennzeichnet, daß die optische Einrichtung (25) aus einem leistungsfähigen optischen Instrument (30), das die Strahlen aufnimmt und überträgt, einer optischen Beobachtungseinrichtung (32) und einem regelbaren Spiegel (31) besteht, der die von dem optischen Instrument (30) übertragenen Strahlen aufnimmt und zum Zielbereich weiterleitet.

7. Einrichtung nach Anspruch 6, dadurch gekennzeichnet, daß das leistungsfähige optische Instrument (30) ein Zoom- objektiv ist.

8. Einrichtung nach einem der Ansprüche 6 oder 7, dadurch gekennzeichnet, daß die optische Beobachtungseinrichtung (32) ein Mikroskop ist, in das der Filter (34), der den Beobachter schützt, wenn der leistungsfähige Strahl (21) ausgelöst ist, eingepaßt ist.

9. Einrichtung nach Anspruch 8, dadurch gekennzeichnet, daß der Filter (34) ständig im Mikroskop (32) befestigt ist, wobei der Filter im Gesichtsfeld des Beobachters angeordnet ist.

10. Einrichtung nach einem der Ansprüche 4-9, dadurch gekennzeichnet, daß eine Blende (35) zwischen den leistungsfähigen Laser (20) und die Kupplung (28) auf der Bahn des durch diesen Laser ausgesendeten Strahls geschaltet ist.

## Claims

1. Laser device of the type comprising at least one power laser (20) capable of emitting radiation of a wavelength situated in the visible spectral field, for producing an action on a target, at least one auxiliary sighting laser (26) capable of emitting radiation (27) of a wavelength situated in the visible spectral field, directed on a zone of said target on which the radiation (21) emitted by said power laser (20) may act, the radiations emitted respectively by the power laser and the sighting laser following a common optical path passing through an optical device (25) which is movable with respect to said target, characterized in that it comprises a filter (34) associated with the optical device (25) and allowing to protect the observer when the power radiation (21) is initiated, and thus allowing him to see the target likewise.

2. Device according to claim 1, characterized in that said power laser (20) is an argon laser.

3. Device according to anyone of claims 1 or 2, characterized in that said auxiliary sighting laser (26) is a helium-neon laser.

4. Device according to anyone of claims 1 or 3, characterized in that said radiations emitted by the power laser (20) and by the auxiliary sighting laser (26) are received by a coupler (28) which aligns said radiations along said common optical path.

5. Device according to anyone of claims 1 to 4, characterized in that said aligned radiations propagate in an optical fiber (24) which emerges in said optical device (25).

6. Device according to anyone of claims 1 to 5, characterized in that said optical device (25) comprises a power optical member (30) receiving and transmitting said radiations, an optical sighting means (32) and an adjustable mirror (31) receiving the radiations transmitted by said optical member and directing them towards said target.

7. Device according to claim 6, characterized in that said power optical member (30) is a zoom.

8. Device according to anyone of claims 6 or 7, characterized in that said optical sighting means (32) is a microscope to which is added said filter (34) for protecting the observer when the power radiation (21) is initiated.

9. Device according to claim 8, characterized in that said filter (34) is permanently fixed to said microscope (32), being placed in the field of vision of the observer.

10. Device according to anyone of claims 4 to 9, characterized in that an obturator (35) is interposed between the power laser (20) and said coupler (28), in the path of the radiation emitted by said laser.

FIG.1A

FIG.1B

FIG.2